(19) Europäisches Patentamt
European Patent Office
Office européen des brevets

(11) Veröffentlichungsnummer: **0 001 089**
**B1**

(12) # EUROPÄISCHE PATENTSCHRIFT

(45) Veröffentlichungstag der Patentschrift: **21.10.81**

(21) Anmeldenummer: **78100782.8**

(22) Anmeldetag: **30.08.78**

(51) Int. Cl.³: **C 07 D 307/32,**
C 07 D 307/94,
C 07 C 69/74,
C 07 C 49/587

(54) **Verfahren zur Herstellung von halogenvinylsubstituierten Tetrahydrofuran-2-onen, bestimmte halogenvinylsubstituierte Tetrahydrofuran-2-one.**

(30) Priorität: **10.09.77 DE 2740849**

(43) Veröffentlichungstag der Anmeldung:
**21.03.79 Patentblatt 79/6**

(45) Bekanntmachung des Hinweises auf die Patenterteilung:
**21.10.81 Patentblatt 81/42**

(84) Benannte Vertragsstaaten:
**BE CH DE FR GB NL**

(56) Entgegenhaltungen:
**DE - A - 2 638 356**
**DE - A - 2 723 447**
**DE - A - 2 813 336**
**US - A - 3 488 732**

**JOURNAL OF THE AMERICAN CHEMICAL SOCIETY, vol. 74 (1952), Seiten 2679—2680**

**TETRAHEDRON LETTERS, 1973, Seiten 923—926**

(73) Patentinhaber: **BAYER AG**
**Zentralbereich Patente, Marken und Lizenzen**
**D-5090 Leverkusen 1, Bayerwerk (DE)**

(72) Erfinder: **Heine, Hans-Georg, Dr.**
**Am Heckerhof 14**
**D-4150 Krefeld (DE)**
Erfinder: **Hübner, Armin, Dr.**
**Mühlenstrasse 26**
**D-4154 Tönisvorst (DE)**
Erfinder: **Hartmann, Willy, Dr.**
**Hohenzollernstrasse 25**
**D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 089 B1

# Verfahren zur Herstellung von halogenvinylsubstituierten Tetrahydrofuran-2-onen, bestimmte halogenvinylsubstituierte Tetrahydrofuran-2-one

Gegenstand der Erfindung ist ein Verfahren zur Herstellung von halogenvinylsubstituierten Tetrahydrofuran-2-onen sowie neue mono-, di- und trihalogenvinylsubstituierte Tetrahydrofuran-2-one.

Halogenvinylsubstituierte Tetrahydrofuran-one sind wertvolle Zwischenprodukte zur Herstellung zum Teil bekannter halogenvinylsubstituierter Cyclopropancarbonsäureester, die als insektizide Verwendung finden. So ist in den Deutschen Offenlegungsschriften 2 623 777 und 2 630 981 ein Weg aufgezeigt, 5-($\beta$,$\beta$-Dichlorvinyl)-4,4-dimethyltetrahydrofuran-2-on in Ester der 2.2-Dimethyl-3-$\beta$,$\beta$-dichlorvinyl)-cyclopropancarbonsäure umzuwandeln. Die dort beschriebenen Verfahren zur Herstellung der für diese Umsetzung benötigten Lactone sind jedoch wenig wirtschaftlich und außerdem auf die Synthese 5-vinylsubstituierter Tetrahydrofuran-2-one beschränkt. Die interessanten isomeren 4-halogenvinylsubstituierten Tetrahydrofuran-2-one, von denen beispielsweise das 5,5-Dimethyl-4-($\beta$,$\beta$-dichlorvinyl)-tetrahydrofuran-2-on aus Pestic. Sci. *1974*, 792 bekannt ist, sind nach DE—A—2 623 777 bzw. 2 630 981 nicht zugänglich.

Weiterhin ist aus der Deutschen Offenlegungsschrift 2 702 222 ein Verfahren zur Herstellung von 4-Halogenvinylsubstituierten Tetrahydrofuran-2-onen bekannt geworden, bei dem ein vinylsubstituiertes Epoxid mit einem Malonsäureester im basischen Medium kondensiert wird und die dabei erhaltenen 3-Alkoxycarbonyl-substituierten Tetrahydrofuran-2-one decarbalkoxyliert werden. Der Nachteil dieses Verfahrens besteht darin, daß danach nur Tetrahydrofuran-2-one zugänglich sind, die in 4-Stellung dihalogenvinylsubstituiert sind. Außerdem sind die Tetrahydrofuran-2-one erst über die entspechenden 3-Alkoxycarbonylsubstituierten Verbindungen zugänglich, was diesen Syntheseweg wenig wirtschaftlich macht.

Die Umsetzung von Cyclobutanonen zu Tetrahydrofuranen war bekannt aus Org. Chemistry 1971, S. 587, J. Am. Chem. Soc. *74*, 2679 (1952) und Tetrahedron Letters (12) 923 (1973).

Es konnte jedoch nicht erwartet werden, daß diese Reaktion ohne unerwünschte Nebenreaktionen auch bei Verbindungen eintritt, die Halogenvinylsubstituenten tragen. So geht aus Modern Synthetic Reactions 1965, S. 119—120 hervor, daß chlorsubstituierte Olefine sehr leicht durch Persäuren oxidiert werden.

Aus US—A—3 488 732 waren halogenvinylsubstituierte Tetrahydrofurane mit nematoziden Eigenschaften bekannt. Ihre nematozide Wirkung ist jedoch vor allem bei niedrigen Aufwandmengen nicht befriedigend. Es wurde eine bestimmte Gruppe halogenvinylsubstituierter Tetrahydrofurane gefunden, die sich durch gute nematozide Wirkung auszeichnet.

1. Es wurde ein Verfahren zur Herstellung von halogenvinylsubstituierten Tetrahydrofuran-2-onen der allgemeinen Formel I gefunden

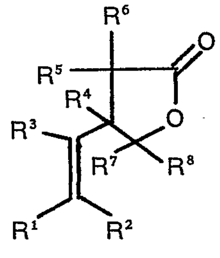

(I)

in welcher $R^1$, $R^2$, $R^3$ und $R^5$ gleich oder verschieden sind und für Wasserstoff, Chlor, Brom, Fluor, Cyan, Alkyl, Alkenyl, Cycloalkyl, sowie für Aryl und Aralkyl stehen und $R^4$, $R^6$, $R^7$ und $R^8$ gleich oder verschieden sind und für Wasserstoff, Alkyl, Alkenyl, Cycloalkyl, sowie für Aryl, ferner für Cyan stehen und außerdem $R^1$ und $R^2$ und/oder $R^3$ und $R^4$ und/oder $R^5$ und $R^6$ oder $R^1$ und $R^3$ und/oder $R^2$ und $R^4$ und/oder $R^5$ und $R^6$ oder $R^1$ und $R^3$ und/oder $R^4$ und $R^5$ oder $R^1$ und $R^2$ und/oder $R^3$ und $R^5$ und/oder $R^4$ und $R^6$ und/oder $R^7$ und $R^8$ mit der Atomen, mit denen sie verknüpft sind, über einen Ring verbunden sind, wobei mindestens einer der Reste $R^1$, $R^2$ oder $R^3$ für Fluor, Chlor oder Brom steht, dadurch gekennzeichnet, daß man ein halogenvinylsubstituiertes Cyclobutanon der allgemeinen Formel II

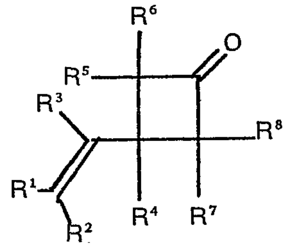

(II)

worin $R^1$—$R^8$ die oben angegebene Bedeutung haben, gegebenenfalls in Gegenwart eines Verdünnungsmittels mit einer Persäure oxydiert.

2. Neue halogenvinylsubstituierte Tetrahydrofuran-2-one der allgemeinen Formel I, in welcher $R^1$—$R^8$ die oben genannte Bedeutung haben, $R^3$ jedoch nicht für Wasserstoff steht.

Zur Durchführung der erfindungsgemäßen Oxidation von Cyclobutanonen der allgemeinen Formel II mit einer organischen Persäure könnnen verschiedene Varianten angewendet werden (s.: Baeyer — Villiger — Oxydation in Modern Synthetic Reaction", W. A. Benjamin Inc., New York (1965), S. 123, "Org. Reactions", Vol. 9, 73 (1957) sowie "Molecular Rearrangements", J. Wiley Inc., New York (1963), Vol. 1, S. 568). So erfolgt die Oxydation beispielsweise zweckmäßig durch Einwirkung einer Persäure, wie Perameisen-, Peressig-, Perpropion-, Trifluorperessig-, Trichlorperessig-, Monopermalein-, Perbenzoe-, m-Chlorbenzoe- oder Monoperphthalsäure, vorzugsweise durch Einwirkung von Peressig-, Perpropion- oder m-Chlorperbenzoesäure. Die organische Persäure kann in situ durch Einwirkung von Wasserstoffperoxid auf die entsprechende organische Säure gebildet werden.

Die Reaktion kann in wäßrigem Medium in Anwesenheit inerter organischer Verdünnungsmittel, wie halogenierte Kohlenwasserstoffe, z.B. Methylenchlorid, Chloroform, Trichloräthylen, Dichloräthylen oder Hexachlorbutadien durchgeführt werden.

Gegebenenfalls kann auf das wäßrige Medium verzichtet werden und nur in einem inerten organischen Verdünnungsmittel gearbeitet werden.

Gemäß einer bevorzugten Ausführungsform des erfindungsgemäßen Verfahrens erfolgt die Oxydation der Cyclobutanone der allgemeinen Formel II in einem gepufferten Medium. Als Puffermittel finden dabei Verwendung: Alkalimetallsalze organischer Säuren, wie Natrium- oder Kaliumformiat, -acetat, -propionat, -butyrat, -oxalat, -citrat oder -tartrat, oder Alkalisalze anorganischer Säuren, wie Natrium- oder Kaliumhydrogencarbonat sowie -carbonat. Dabei wird Natriumacetat in einem wäßrigen, organischen Verdünnungsmedium, Natriumhydrogencarbonat in einem organischen Verdünnungsmittel bevorzugt.

Die Geschwindigkeit der Umsetzung nach dem erfindungsgemäßen Verfahren hängt von verschiedenen Faktoren ab, z.B. der Temperatur, den besonderen eingesetzten Reaktionspartnern und dem verwendeten Lösungsmittel. Im allgemeinen erfolgt die Umsetzung bei 20°C oder wenig darüber oder darunter.

Zur Erzielung optimaler Ausbeuten an Reaktionsprodukt, insbesondere bei größeren Ansätzen, kann die die organische Persäure enthaltende Lösung vorteilhaft zu der, das Cyclobutanon enthaltenden Lösung getropft werden, wobei die Temperatur beispielsweise durch Kühlung auf einem bestimmten Wert (z.B. 20°C) gehalten werden kann. Zur Aufarbeitung zersetzt man überschüssige Persäure in an sich bekannter Weise mit einem Reduktionsmittel und trennt das entstandene halogenvinylsubstituierte Tetrahydrofuran-2-one durch Kristallisieren, Destillieren oder Chromatographieren ab.

Die zur Durchführung des erfindungsgemäßen Verfahrens verwendbaren halogenvinylsubstituierten Cyclobutanone der allgemeinen Formel II sind zum Teil bekannt. Sie werden nach einem nicht zum Stand der Technik gehörenden Verfahren hergestellt, indem man ein N.N-disubstituiertes Carbonsäureamid der allgemeinen Formel III

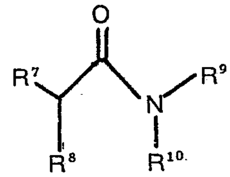

(III)

worin $R^7$ und $R^8$ die voranstehend genannte Bedeutung haben, $R^9$ und $R^{10}$ gleich oder verschieden sein können und für Alkyl, Cycloalkyl, Alkenyl, Aryl, Aralkyl stehen und ferner $R^9$ und $R^{10}$ mit den Atomen, mit denen sie verknüpft sind, einen Ring bilden, mit einem anorganischen Säurehalogenid, vorzugsweise Phosgen oder Thionylchlorid, umsetzt und anschliessend mit einem tert. Amin und einem Olefin der allgemeinen Formel IV

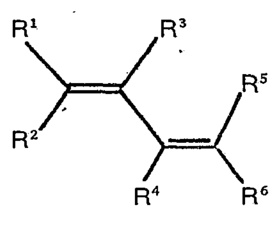

(IV)

worin $R^1$—$R^6$ die voranstehend genannte Bedeutung haben, und einer Lewis-Säure zur Reaktion bringt sowie anschließend hydrolysiert und gegebenenfalls halogeniert (vgl. Deutsche Patentanmeldung P 2 715 336.2).

Für die Umsetzung geeignete halogenvinylsubstituierte Cyclobutanone der allgemeinen Formel II sind:

2,2-Dimethyl-3-(α-methyl-β,β-dichlorvinyl)-cyclobutanon
2,2-Diäthyl-3-(α,β-dichlorvinyl)-cyclobutanon
2,2-Dimethyl-3-(α,β,β-trifluorvinyl)-cyclobutanon
2,2-Diäthyl-3-(α,β,β-trichlorvinyl)-cyclobutanon
2,2-Dimethyl-3-(α,β-dichlorvinyl)-cyclobutanon
2,2-Dimethyl-3-(β,β-dibromvinyl)-cyclobutanon
2,2-Dimethyl-3-(α-fluor-β,β-dichlorvinyl)-cyclobutanon
2,2-Dimethyl-3-(β-chlorvinyl)-cyclobutanon
2,2,3-Trimethyl-3-(α,β,β-trifluorvinyl)-cyclobutanon
2,2-Dimethyl-3-(β,β-difluorvinyl)-cyclobutanon
2-Äthyl-2-methyl-3-(β,β-dichlorvinyl)-cyclobutanon
2-Äthyl-2-methyl-3-(α,β,β-trichlorvinyl)-cyclobutanon
2-Äthyl-2,3-dimethyl-3-(α,β,β-trichlorvinyl)-cyclobutanon
2,2-Diäthyl-3-(β,β-dibromvinyl)-cyclobutanon
2-Äthyl-2-methyl-3-(β,β-dibromvinyl)-cyclobutanon
2-Äthyl-2-methyl-3-(α-fluor-β,β-dichlorvinyl)-cyclobutanon
2,2-Dimethyl-3-(α-äthyl-β,β-dichlorvinyl)-cyclobutanon
2-Äthyl-2,3-dimethyl-3-(β,β-dichlorvinyl)-cyclobutanon
4-(β,β-dichlorvinyl)-spiro[3,5]-nonanon-2
2,2-Dimethyl-3-(α,β-dibromvinyl)-cyclobutanon
4-(α,β,β-Trichlorvinyl)-spiro[3,5]-nonanon-2
4-(β,β-Dibromvinyl)-spiro[3,5]-nonanon-2
2,2-Dimethyl-3-(β-brom-β-chlorvinyl)-cyclobutanon
2,2-Dimethyl-4-äthyl-3-(β, β-dichlorvinyl)-cyclobutanon
2,2,4-Trimethyl-3-(α,β-dibromvinyl)-cyclobutanon
2,2-Dimethyl-4-n-butyl-3-(β,β-dichlorvinyl)-cyclobutanon
2-Methyl-3-(α,β,β-trichlorvinyl)-cyclobutanon

In 4-Stellung durch Chlor oder Brom substituiertes

2,2-Dimethyl-3-(α-methyl-β,β-dichlorvinyl)-cyclobutanon
2,2-Diäthyl-3-(α,β-dichlorvinyl)-cyclobutanon
2,2-Dimethyl-3-(α,β,β-trifluorvinyl)-cyclobutanon
2,2-Diäthyl-3-(α,β,β-trichlorvinyl)-cyclobutanon
2,2-Dimethyl-3-(α,β-dichlorvinyl)-cyclobutanon
2,2-Dimethyl-3-(β,β-dibromvinyl)-cyclobutanon
2,2-Dimethyl-3-(α-fluor-β,β-dichlorvinyl)-cyclobutanon
2,2-Dimethyl-3-(β-chlorvinyl)-cyclobutanon
2,2,3-Trimethyl-3-(α,β,β-trifluorvinyl)-cyclobutanon
2,2-Dimethyl-3-(β,β-difluorvinyl)-cyclobutanon
2-Äthyl-2-methyl-3-(β,β-dichlorvinyl)-cyclobutanon
2-Äthyl-2-methyl-3-(α,β,β-trichlorvinyl)-cyclobutanon
2-Äthyl-2,3-dimethyl-3-(α,β,β-trichlorvinyl)-cyclobutanon
2,2-Diäthyl-3-(β,β-dibromvinyl)-cyclobutanon
2-Äthyl-2-methyl-3-(β,β-dibromvinyl)-cyclobutanon
2-Äthyl-2-methyl-3-(α-fluor-β,β-dichlorvinyl)-cyclobutanon
2,2-Dimethyl-3-(α-äthyl-β,β-dichlorvinyl)-cyclobutanon
2-Äthyl-2,3-dimethyl-3-(β,β-dichlorvinyl)-cyclobutanon
2,2-Dimethyl-3-(α,β-dibromvinyl)-cyclobutanon
2,2-Dimethyl-3-(β-brom-β-chlorvinyl)-cyclobutanon
2,2-Dimethyl-4-äthyl-3-(β,β-dichlorvinyl)-cyclobutanon
2,2,4-Trimethyl-3-(α,β-dibromvinyl)-cyclobutanon
2,2-Dimethyl-4-n-butyl-3-(β,β-dichlorvinyl)-cyclobutanon
2-Methyl-3-(α,β,β-trichlorvinyl)-cyclobutanon
2,2-Dimethyl-3-(β,β-dichlorvinyl)-cyclobutanon
2,2-Dimethyl-3-(α,β,β-trichlorvinyl)-cyclobutanon
2,2,3-Trimethyl-3-(α,β,β-trichlorvinyl)-cyclobutanon
2,2-Di-n-propyl-3-(α,β,β-trichlorvinyl)-cyclobutanon
2-2-Dimethyl-3-(α-cyan-β,β-dichlorvinyl)-cyclobutanon
2,2-Dimethyl-3-n-butyl-3-(β,β-dichlorvinyl)-cyclobutanon
2,2-Dimethyl-3-(β,β-dicyanvinyl)-cyclobutanon
2,3-Dimethyl-3-(β,β-dibromvinyl)-cyclobutanon

2,2-Dimethyl-3-(β,β-dibromvinyl)-4-n-butyl-cyclobutanon
2,2-Di-n-butyl-3-methyl-3-(α-chlor-β-cyanvinyl)-cyclobutanon
2,3-Diäthyl-3-(β,β-dichlorvinyl)-4-cyclohexyl-cyclobutanon
2,3-Dimethyl-2-chlor-3-(α,β,β-trichlorvinyl)-cyclobutanon
2-Methyl-2-phenyl-3-(β,β-dichlorvinyl)-cyclobutanon
2,2-Dimethyl-3-(β-chlor-β-phenylvinyl)-cyclobutanon
2,2-Dimethyl-3-(α,β,β-trichlorvinyl)-4-benzyl-cyclobutanon

In 3-Stellung halogenierte spirocyclische Cyclobutanone sind:

4-(β,β-Dichlorvinyl)-spiro[5,3]nonanon-2
4-(α,β,β-Trichlorvinyl)-spiro[5,3]nonanon-2
4-(β,β-Dibromvinyl)-spiro[5,3]nonanon-2
4-(β,β-Dichlorvinyl)-spiro[4,3]octanon-2
4-(β,β-Dichlorvinyl)-3-methyl-spiro[5,3]nonanon-2
4-(α,β-Dichlorvinyl)-spiro[5,3]nonanon-2
4-(α,β,β-Trifluorvinyl)-spiro[5,3]nonanon-2

Die nach den erfindungsgemäßen Verfahren leicht zugänglichen halogenvinylsubstituierten Tetrahydrofuran-2-one der allgemeinen Formel I sind Zwischenprodukte zur Herstellung insektizider Wirkstoffe. So läßt sich z.B. das 4-(β,β-Dichlorvinyl)-5,5-dimethyl-tetrahydrofuran-2-on mit Thionylchlorid glatt zum 3-(β.β-Dichlorvinyl)-4-chlor-4-methyl-valeriansäurechlorid öffnen, das mit m-Phenoxybenzylalkohol zum entsprechenden Ester umgesetzt wird, welcher anschließend in Gegenwart von Natriumalkoholat zu dem insektiziden Wirkstoff 2.2-Dimethyl-3-(β,β-dichlorvinyl)-cyclopropancarbonsäure-m-phenoxybenzylester cyclisiert wird.

Die folgenden Beispiele erläutern das erfindungsgemäße Verfahren.

Beispiel 1

Zu einer Lösung von 41,8 g (0,22 Mol) 2.2-Dimethyl-3-(β.β-dichlorvinyl)-cyclobutanon in 500 ml Chloroform werden bei 20°C 26,0 g (0,3 Mol) Natriumhydrogenbicarbonat und 52,0 g (0,3 Mol) m-Chlorperbenzoesäure gegeben. Nach 15 stdg. Stehen bei 20°C werden 10 ml 10 proz. wäßrige Natriumhydrogensulfit-Lösung hinzugefügt und 1 h gerührt. Die organische Phase wird abgetrennt, über wasserfreiem Natriumsulfat getrocknet und eingedampft. Der kristalline Rückstand (43,2 g) besteht einheitlich aus 4-(β.β-Dichlorvinyl-5.5-dimethyl-tetrahydrofuran-2-on vom Schmp. 117—118°C (aus Benzol/n-Hexan).

Beispiel 2

22,7 g (0,1 Mol) 2.2-Dimethyl-3-(α.β.β-trichlorvinyl)-cyclobutanon, 13,0 g (0,15 Mol) Natriumhydrogencarbonat und 26,0 g (0,15 Mol) m-Chlorperbenzoesäure werden in 300 ml Chloroform unter Kühlen bei 20°C gerührt. Nach 23 h werden 10 ml 10 proz. wäßrige Natriumhydrogensulfit-Lösung zugesetzt und 1 h nachgerührt. Abtrennen der organischen Pase, Waschen mit gesättigter wäßriger Natriumhydrogencarbonat-Lösung, Trocknen der organischen Phase über Natriumsulfat und Eindampfen liefern 23,1 g kristallines 4-(α.β.β-Trichlorvinyl)-5.5-dimethyltetrahydrofuran-2-on von Schmp. 68—70°C (aus n-Hexan).

Beispiel 3

Eine Lösung von 79,3 g (0,5 Mol) 2.2-Dimethyl-3-(β-chlorvinyl)-cyclobutanon (Stereoisomerengemisch) in 1500 ml Chloroform wird unter Kühlen mit 63,0 g (0,75 Mol) Natriumhydrogencarbonat und 129,3 g (0,75 Mol) m-Chlorperbenzoesäure bei 20°C unter Rühren versetzt. Nach 48 h werden 500 ml Chloroform zugesetzt. Aufarbeiten, wie in Beispiel 2 angegeben, liefert 92,2 g gelbliches Öl, das fraktionierend destilliert wird. 59,8 g farbloses 4-(β-Chlorvinyl)-5.5-dimethyl-tetrahydrofuran-2-on vom $Sdp._{11}$ 139°C $n_D^{20} = 1{,}4823$ werden erhalten.

Beispiel 4

30,6 g (0,1 Mol) 2.2-Dimethyl-3-(α.β.β-trichlorvinyl)-4-brom-cyclobutanon (einheitliches Stereoisomer) und 12,6 g (0,15 Mol) Natriumhydrogencarbonat in 200 ml Chlorform werden unter Kühlen bei 20°C mit 100 ml Chlorform, die 0,12 Mol Perbenzoesäure enthalten, tropfenweise versetzt. Nach 48 h wird der Ansatz, wie in Beispiel 2 beschrieben, aufgearbeitet. 31,7 g kristallines 3-Brom-4-(α.β.β-trichlorvinyl)-5.5-dimethyl-tetrahydrofuran-2-on vom Schmp. 110—111°C (aus Äther/n-Hexan) werden erhalten.

Beispiel 5

Einer Lösung von 6,66 g (0,029 Mol) 3-(β.β-Dichlorvinyl)-spiro[3,5]nonan-1-on in 200 ml Chloroform werden bei 20°C nacheinander 4,50 g (0,05 Mol) Natriumhydrogencarbonat und 5,20 g (0,03 Mol) m-Chlorperbenzoesäure zugesetzt. Nach 24 stdg. rühren bei 20°C wird, wie in Beispiel 2 beschrieben, aufgearbeitet. Chromatographie des kristallinen Rohprodukts (7,0 g) an Kieselgel mit Benzol liefert 6,2 g farbloses 1-Oxa-4-(β.β-dichlorvinyl)-spiro[4,5]decan-2-on vom Schmp. 98°C (aus Äther/n-Hexan).

Beispiel 5a

Zu einer Lösung von 5.0 g (0,022 Mol) 3-(β.β-Dichlorvinyl)-spiro[3,5]nonan-1-on in 50 ml Eisessig werden bei 20°C 10 ml 30 proz. Wasserstoffperoxid (0,09 Mol) getropft. Nach 24 stdg. Rühren bei 20°C wird der Ansatz auf Eis gegossen und der Niederschlag abfiltriert. 5.0 g kristallines 1-Oxa-4-(β.β-dichlorvinyl)-spiro[4,5]decan-2-on vom Schmp. 98°C werden erhalten.

Das folgende Beispiel zeigt die Herstellung eines insektiziden Wirkstoffes aus nach dem erfindungsgemäßen Verfahren erhältlichen Lactonen:

Beispiel 6

20,9 g (0,1 Mol) 4-(β.β-Dichlorvinyl)-5.5-dimethyl-tetrahydrofuran-2-on werden in 80 ml Toluol gelöst. Nach Zugabe von 20 ml Thionylchlorid erhitzt man 12 h zum Sieden. Nach dem Abkühlen werden 22 g (0,11 Mol) 3-Phenoxybenzylalkohol zugetropft, sowie 3 g Tetrabutylphosphoniumchlorid zugegeben. Dann tropft man bei Raumtemperatur 30 g 50%ige Kalilauge zu. Man rührt 2 h bei Raumtemperatur nach, gibt Eiswasser zu und stellt neutral. Nach Abtrennen der Toluolphase wird das Toluol abdestilliert und der Rückstand an Kieselgel mit Benzol als Laufmittel gereinigt. Man erhält ein Produkt, das zu 95% aus trans-2,2-Dimethyl-3-(2′,2′-dichlorvinyl)-cyclopropancarbonsäure-3-phenoxybenzylester ($n^D_{20} = 1,5616$) besteht.

Die folgenden Beispiele zeigen die Herstellung der als Ausgangsprodukte für das erfindungsgemäße Verfahren zu verwendenden Cyclobutanone:

Beispiel 7a

Herstellung des Amidchlorids des Isobuttersäuredimethylamids.

In ein Rührgefäss, versehen mit Rührer, Rückflusskühler, Tropfrichter und Gaseinleitungsrohr, gibt man die Lösung von 345.0 g (3.0 Mol) Isobuttersäuredimethylamid in 2000 ml Methylenchlorid und leitet unter Kühlen bei 0°C 330.0 g (3.3 Mol) Phosgen unter Rühren ein. Man lässt die Lösung auf 20—25°C erwärmen und destilliert nach Stehen über Nacht (15 Stunden) nichtumgesetztes Phosgen zusammen mit etwa 1/3 des als Lösungsmittel verwendeten Methylenchlorides ab. Den Rückstand verdünnt man durch Zugabe von Methylenchlorid auf 2100 ml.

Beispiel 7b

Herstellung von 2.2-Dimethyl-3-(α,β,β-trichlorvinyl)-cyclobutanon.

Zu 350 ml der gemäss Beispiel 7a bereiteten Lösung des Amidchlorids des Isobuttersäuredimethylamids in Methylenchlorid tropft man unter Kühlen und Rühren bei 20°C 50.0 g (0.5 Mol) Triäthylamin in 100 ml Methylenchlorid und erhitzt anschließend 1 Stunde zum Rückfluss. Darauf fügt man bei 10°C 75.0 g (0.55 Mol) Zinkchlorid hinzu und tropft 79.0 g (0.5 Mol) 1.1.2-Trichlorbutadien innerhalb von 60 Min. in die Reaktionslösung. Nach 5-stündigem Erhitzen unter Rückfluss versetzt man sie mit 400 ml Wasser und rührt über Nacht (15 Stunden). Trennen der Phasen, Trocknen der organischen Phase über Natriumsulfat und fraktionierendes Destillieren liefern 10.5 g 1.1.2-Trichlorbutadien, 11.5 g Isobuttersäuredimethylamid und 66.8 g (59%, bezogen auf eingesetztes Isobuttersäuredimethylamid) Keton vom $Sdp._{10}$ 112—117°C $n_D^{20}$ 1.509.

Beispiel 8

Herstellung von 3-(β,β-Dichlorvinyl)-spiro[3,5]nonanon-1

In die Lösung von 77.5 g (0.5 Mol) Cyclohexancarbonsäuredimethylamid und 300 ml Chlorbenzol leitet man bei 20°C 55.0 g (0.55 Mol) Phosgen. Nach 5-stündigem rühren bei 30—40°C zieht man nichtumgesetztes Phosgen unter vermindertem Druck ab und tropft anschliessend 55.0 g (0.55 Mol) Triäthylamin bei 20°C zu. Man erhitzt 1 Stunde auf 40—50°C, kühlt ab, fügt bei 20°C 75.0 g (0.55 Mol) Zinkchlorid zu und tropft anschliessend 61.5 g (0.5 Mol) 1.1-Dichlorbutadien in 50 ml Chlorbenzol zu. Nach 6-stündigem Erwärmen auf 40—50°C arbeitet man, wie in Beispiel 7b beschrieben, auf. Fraktionierende Destillation liefert 69.6 g Kristalle vom Schmp. 58—60°C (aus n-Hexan).

Beispiel 9

Herstellung von 2.2-Dimethyl-3-(β-chlorvinyl)-cyclobutanon

Zu 350 ml der gemäss Beispiel 7a bereiteten Lösung des Amidchlorids des Isobuttersäuredimethylamids in Methylenchlorid gibt man unter Kühlen und Rühren bei 0°C 44,25 g (0,5 Mol) 1-Chlorbutadien (Isomerengemisch) und darauf 50.0 g (0.5 Mol) Triäthylamin in 100 ml Methylenchlorid. Nach Erwärmen auf 20°C gibt man portionsweise unter Kühlen 75.0 g (0.55 Mol) Zinkchlorid hinzu und erhitzt anschliessend 6 Stunden zum Rückfluss. Nach Zugabe von 250 ml Wasser und üblicher Aufarbeitung erhält man 54.2 g (68% bezogen auf eingesetztes Isobuttersäuredimethylamid) vom $Sdp._{11-12}$ 81—85°C $n_D^{20}$ 1.4759.

**Patentansprüche**

1. Verfahren zur Herstellung von halogenvinylsubstituierten Tetrahydrofuran-2-onen der allgemeinen Formel I

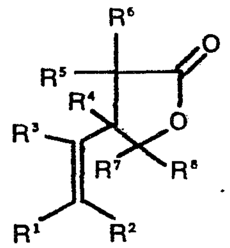

(I)

in welcher $R^1$, $R^2$, $R^3$ und $R^5$ gleich oder verschieden sind und für Wasserstoff, Chlor, Brom, Fluor, Cyan, Alkyl, Alkenyl, Cycloalkyl, sowie für Aryl und Aralkyl stehen und $R^4$, $R^6$, $R^7$ und $R^8$ gleich oder verschieden sind und für Wasserstoff, Alkyl, Alkenyl, Cycloalkyl, sowie für Aryl, ferner für Cyan stehen und außerdem $R^1$ und $R^2$ und/oder $R^3$ und $R^4$ und/oder $R^5$ und $R^6$ oder $R^1$ und $R^3$ und/oder $R^2$ und $R^4$ und/oder $R^5$ und $R^6$ oder $R^1$ und $R^3$ und/oder $R^4$ und $R^5$ oder $R^1$ und $R^2$ und/oder $R^3$ und $R^5$ und/oder $R^4$ und $R^6$ und/oder $R^7$ und $R^8$ mit den Atomen, mit denen sie verknüpft sind, über einen Ring verbunden sind, wobei mindestens einer der Reste $R^1$, $R^2$ oder $R^3$ für Fluor, Chlor oder Brom steht, dadurch gekennzeichnet, daß man ein halogenvinylsubstituiertes Cyclobutanon der allgemeinen Formel II

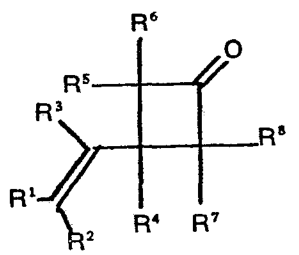

(II)

worin $R^1$—$R^8$ die oben angegebene Bedeutung haben, gegebenenfalls in Gegenwart eines Verdünnungsmittels mit einer Persäure oxydiert.

2. Halogenvinylsubstituierte Tetrahydrofuran-2-one der allgemeinen Formel I, in welcher $R^1$—$R^8$ die oben genannte Bedeutung haben, jedoch mit der Maßgabe, daß $R^3$ nicht für Wasserstoff steht.

3. Halogenvinylsubstituiertes Tetrahydrofuranon der Formel

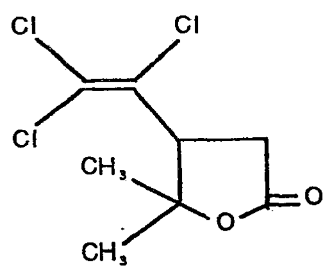

**Claims**

1. Process for the preparation of halogenovinyl-substituted tetrahydrofuran-2-ones of the general formula I

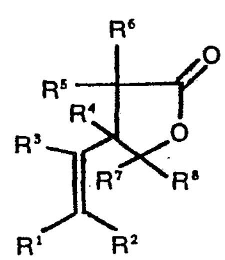

(I)

in which $R^1$, $R^2$, $R^3$ and $R^5$ are identical or different and represent hydrogen, chlorine, bromine, fluorine, cyano, alkyl, alkenyl, cycloalkyl, as well as aryl and aralkyl and $R^4$, $R^6$, $R^7$ and $R^8$ are identical or different and represent hydrogen, alkyl, alkenyl, cycloalkyl, as well as aryl, furthermore cyano and in addition $R^1$ and $R^2$ and/or $R^3$ and $R^4$ and/or $R^5$ and $R^6$ or $R^1$ and $R^3$ and/or $R^2$ and $R^4$ and/or $R^5$ and $R^6$ or $R^1$ and $R^3$ and/or $R^4$ and $R^5$ or $R^1$ and $R^2$ and/or $R^3$ and $R^5$ and/or $R^4$ and $R^6$ and/or $R^7$ and $R^8$, with the atoms to

which they are linked, are bonded via a ring, at least one of the radicals $R^1$, $R^2$ or $R^3$ representing fluorine, chlorine or bromine characterised in that a halogenovinyl-substituted cyclobutanone of the general formula II

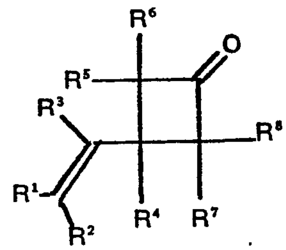

(II)

in which $R^1$—$R^8$ have the meaning indicated above, is oxidized with a peracid optionally in the presence of a diluent.

2. Halogenovinyl-substituted tetrahydrofuran-2-ones of the general formula I in which $R^1$—$R^8$ have the meaning indicated above, however with the proviso that $R^3$ does not represent hydrogen.

3. Halogenovinyl-substituted tetrahydrofuranone of the formula

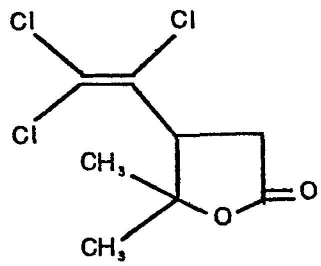

**Revendications**

1. Procédé de production de tétrahydrofuranne-2-ones à substitution halogénovinylique de formule générale I:

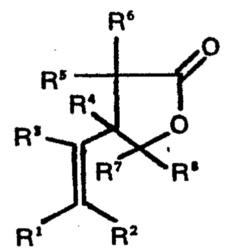

(I)

dans laquelle: $R^1$, $R^2$, $R^3$ et $R^5$ sont égaux ou différents et représentent de l'hydrogène, du chlore, du brome, du fluor, un groupe cyano, alkyle, alcényle, cycloalkyle, ainsi que aryle et aralkyle, et $R^4$, $R^6$, $R^7$ et $R^8$ sont égaux ou différents et représentent de l'hydrogène, un groupe alkyle, alcényle, cycloalkyle ainsi que aryle, de même qu'un groupe cyano, et en outre $R^1$ et $R^2$ et/ou $R^3$ et $R^4$ et/ou $R^5$ et $R^6$ ou $R^1$ et $R^3$ et/ou $R^2$ et $R^4$ et/ou $R^5$ et $R^6$ ou $R^1$ et $R^3$ et/ou $R^4$ et $R^5$ ou $R^1$ et $R^2$ et/ou $R^3$ et $R^5$ et/ou $R^4$ et $R^6$ et/ou $R^7$ et $R^8$ sont liés par l'intermédiaire d'un noyau avec les atomes avec lesquels ils sont attachés, l'un au moins des restes $R^1$, $R^2$ ou $R^3$ représentant du fluor, du chlore ou du brome, caractérisé en ce qu'on oxyde avec un peracide, éventuellement en présence d'un agent diluant, une cyclobutanone à substitution halogénovinylique, de formule générale II:

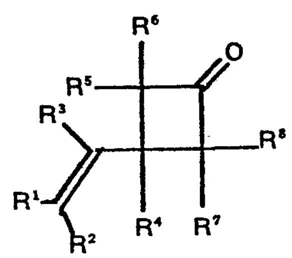

(II)

dans laquelle: $R^1$ à $R^8$ ont la définition indiquée ci-dessus.

2. Des tétrahydrofuranne-2-ones à substitution halogénovinylique de formule générale I dans laquelle $R^1$—$R^8$ ont la définition donnée ci-dessus, mais à condition que $R^3$ no soit pas de l'hydrogène.

3. La tétrahydrofurannone à substitution halogénovinylique de formule: